# EUROPEAN PATENT SPECIFICATION

(11) **EP 1 839 677 B1**
(45) Date of publication and mention of the grant of the patent: **15.12.2010**
(21) Application number: 05781799.1
(22) Date of filing: 02.09.2005
(51) Int. Cl.: A61K 49/22, A61K 9/127, A61K 31/685, A61P 35/00, A61K 41/00

(54) **ADJUVANT OF FLUOROCARBON EMULSIONS FOR HIFU THERAPY AND THE USE THEREOF**
ADJUVANS VON FLUOROCARBON-EMULSIONEN ZUR HIFU-THERAPIE UND VERWENDUNG DAVON
ADJUVANT D'EMULSION FLUOROCARBONES POUR THERAPIES HIFU ET SON UTILISATION

(30) Priority: 10.01.2005 CN 200510000343
(43) Date of publication of application: 03.10.2007
(73) Proprietor: CHONGQING HAIFU(HIFU)TECHNOLOGY CO., LTD, Chongqing 401121 (CN)
(72) Inventor: WANG, Zhibiao, Yubei District, Chongqing 401121 (CN); LI, Faqi, Yubei District, Chongqing 401121 (CN); XIAO, Ziwen, Yubei District, Chongqing 401121 (CN); XIAO, Yanbing, Yubei District, Chongqing 401121 (CN); LIU, Liping, Yubei District, Chongqing 401121 (CN); WANG, Zhilong, Yubei District, Chongqing 401121 (CN)
(74) Representative: Kampfenkel, Klaus
(86) International application number: PCT/CN2005/001391
(87) International publication number: WO 2006/072200

(56) References cited:
- EP-A- 1 323 434
- CN-A- 1 148 814
- CN-A- 1 213 972
- CN-A- 1 404 878
- DE-A1- 4 127 442
- JP-A- 58 032 829
- US-A- 4 767 610
- US-A- 4 987 154
- US-B1- 6 551 576
- MCDANNOLD N. ET AL.: "Heat-activated liposomal MR contrast agent:initial in vivo results in rabbit liver and kidney" RADIOLOGY, vol. 744, 2004, pages 743-752, XP007903928
- GENNARO A. R. ET AL.: "Remington: the Science and Practice of Pharmacy 20th edition." 2000, LINPINNCOTT WILLIAMS AND WILKINS , BALTIMORE USA , XP007903936 * page 847; tables 44-4 *
- SHU-QUN CHENG ET AL: "Iodized oil enhances the thermal effect of high-intensity focused ultrasound on ablating experimental liver cancer" JOURNAL OF CANCER RESEARCH AND CLINICAL ONCOLOGY, SPRINGER-VERLAG, BE, vol. 123, 1997, pages 639-644, XP007903958 ISSN: 1432-1335
- HARVEY C J ET AL: "ADVANCES IN ULTRASOUND" CLINICAL RADIOLOGY, LIVINGSTONE, HARLOW,, GB, vol. 57, no. 3, March 2002 (2002-03), pages 157-177, XP009071168 ISSN: 0009-9260
- JI XIAOJUAN ET AL.: 'the use of untrasound contrast-agent in HIFU against tumors' CHINA J ULTRASONOGR vol. 12, no. 12, December 2003, pages 748 - 750, XP008091713
- WANG D. ET AL.: 'experimental study of tumor in the rabbit brain enhancement imaging using a self-made liposome perfluorocarbon contrast agent' J ULTRASOUND IN CLIN MED vol. 5, no. 3, June 2003, pages 129 - 130, XP008097824

## Description

### FIELD OF THE PRESENT INVENTION

The present invention is related to the fields of medicine and medical treatment, specifically to the field of ultrasound treatment, and more particularly to a fluoro-carbon emulsion enhancement agent for HIFU treatment, which can increase acoustic energy deposition at the target location during HIFU treatment, and use thereof.

### BACKGROUND OF THE PRESENT INVENTION

High-intensity focused ultrasound (HIFU) as a new technique to treat tumors and other diseases has already been recognized in clinical applications. HIFU employs focused ultrasound, which provides continuous, high-intensity ultrasound energy at the focus, resulting in instantaneous thermal effects (65-100°C), cavitation effects, mechanical effects and sonochemical effects, to selectively cause coagulative necrosis at the focus, and prevent tumors from proliferation, invasion and metastasis.

Xiaojuan Ji et al. disclose the use of ultrasound contrast agents in HIFU treatment, wherein the contrast agents are microbubbles ("The use of ultrasound contrast-agent in HIFU against tumors", China J. Ultrasonogr., vol. 12, no. 12, December 2003, pages 748-750).

C. J. Harvey et al. also report on the application of gas containing microbubbles for HIFU therapy and their complex interactions with ultrasound waves ("Advances in ultrasound", Clinical Radiology, Livingstone, Harlow, GB, vol. 57, no. 3, March 2003, pages 155-157, XP009071168,ISSN 0009-9260).

It was demonstrated that the acoustic energy was attenuated exponentially as the transmission distance increased during the ultrasound transmission within the body (Baoqin Liu et al., Chinese Journal of Ultrasound in Medicine, 2002, 18(8):565-568). In addition, the energy during ultrasound transmission in soft tissues was attenuated due to tissue absorption, scattering, refraction, diffraction and the like, among which tissue absorption and scattering are mainly responsible for the energy loss (Ruo Feng and Zhibiao Wang as editors in chief, Practical Ultrasound Therapeutics, Science and Technology Reference Publisher of China, Beijing, 2002.14). Therefore, when the HIFU treatment is used to treat deep-seated and large-sized tumors, the acoustic energy transmitted to the target would be relatively low. Thus, therapeutic efficiency would decrease and the treatment time would be prolonged due to the acoustic energy attenuation.

Of course, although the transmitting power of the therapeutic transducer might be increased in order to improve the therapeutic efficiency, the normal tissue along the pathway of the ultrasound transmission is more likely to be burned in a high-intensity ultrasound environment.

In addition, at present, when the HIFU technique is clinically applied to a hepatic tumor that is blocked by the ribs in the pathway of the ultrasound transmission, the ribs are usually removed in order to increase the energy deposition at the target location, shorten the treatment time and improve therapeutic effects. Thus the noninvasiveness of HIFU treatment cannot be ensured, which is undesirable for the patients and doctors.

The above problems have disadvantageously limited the use of the HIFU treatment as a technique for clinical practice. Therefore, the technical problems with respect to increasing the energy deposition at target location, effectively treating the deep-seated tumors without damaging the surrounding normal tissue in the acoustic pathway, and treating a hepatic tumor that is blocked by the ribs without removal of the ribs, need to be solved urgently.

### SUMMARY OF THE INVENTION

One objective of the present invention is to provide a fluoro-carbon emulsion enhancement agent for HIFU treatment, which can enhance acoustic energy deposition at the target tissue during HIFU treatment.

Another objective of the present invention is to provide a method for enhancing acoustic energy deposition at the target location during HIFU treatment using the fluoro-carbon emulsion enhancement agent of the present invention for HIFU treatment.

A further objective of the present invention is to provide use of a fluoro-carbon emulsion enhancement agent for HIFU treatment to enhance the effectiveness of HIFU treatment.

In order to achieve the above objectives, in one embodiment, the present invention provides an enhancement agent for HIFU treatment. The enhancement agent of the present invention is a substance that can enhance acoustic energy absorption at the target location to be treated with HIFU after its administration to a biological body, i.e. a substance that can be used to reduce the acoustic energy needed to cause lesions of a target tissue (tumor and non-tumor tissue) per unit volume of the tissue during HIFU treatment. In the present invention, the types of the substances used as the enhancement agents for HIFU treatment are not particularly limited, as long as the substances are fluoro-carbon emulsions and can change the acoustic environment of the target tissue and promote therapeutic acoustic energy absorption and deposition at the target tissue.

As used herein, the term "lesion" refers to the substantial change in the physiological state of a tumor or normal tissue, generally refers to the coagulative necrosis of a tumor or normal tissue. Energy efficiency factor (EEF) can be used to quantify the acoustic energy needed to cause lesions of a target tissue per unit volume of the tissue. EEF is presented by the expression of *EEF* = ^{η*Pt*}/*_{V}* (unit: J/mm³), and refers to the acoustic energy needed to cause lesions of a tumor or normal tissue per unit volume of the tissue, wherein, η refers to the focusing coefficient of a HIFU transducer, which reflects the ultrasound energy focusing capacity of the transducer, here η=0.7; P refers to the total acoustic power of a HIFU source (unit: W), t refers to the total time of HIFU treatment (unit: s); and V refers to the volume of HIFU-induced lesions (unit: mm³). A substance that greatly decreases the EEF of the target tissue after its administration is more suitable to be used as the enhancement agent for HIFU treatment according to the present invention.

The enhancement agent for HIFU treatment greatly decreases the EEF of the target tissue after its administration. As a result, the ratio between the EEF of the target tissue measured before the administration of the enhancement agent (i.e. EEF_{(base)}) and the EEF of the target tissue measured after the administration of the enhancement agent (i.e. EEF₍ₘₑₐₛᵤᵣₑₘₑₙₜ₎) is more than 1, preferably more than 2, and more preferably over 4. The upper limit of the ratio is not particularly limited and a higher ratio is preferred.

Specifically, the enhancement agent for HIFU treatment of the present invention comprises a discontinuous phase comprised of a core encapsulated by a membrane-forming material and a continuous phase comprising of aqueous medium. The discontinuous phase is uniformly dispersed in the continuous phase and the particle size of the discontinuous phase ranges from 0.1-1 µm ; the amount of the membrane-forming material in the enhancement agent is 0.1-100g/L, preferably 1-50g/L and more preferably 5-20g/L; the core is comprised of a liquid that undergoes a liquid-gas phase transition at 38-100°C (namely, the liquid can turn into gas within an animal body or human body during HIFU treatment), and the amount of the core material in the enhancement agent is 5-200ml/L, preferably 10-100ml/L, and more preferably 20-80ml/L.

In the above embodiment of the present invention, the membrane-forming material includes: lipids, such as 3-sn-phosphatidylcholine, 1,2-dipalmitoyl-sn-glycero-3-phosphatidylglycerol sodium salt, 1,2-distearoyl-sn-glycero-3-phosphatidylcholine, sodium 1,2-dipalmitoyl-sn-glycero-3-phosphatidate, 1,2-dipalmitoyl-sn-glycero-3-phosphatidylcholine, phosphatidylserine and hydrogenated phosphatidylserine, cholesterol, and glycolipide; saccharides, including, for example, glucose, fructose, sucrose, starch and the degradation products thereof; proteins, such as albumin, globulin, fibrinogen, fibrin, hemoglobin, and the degradation products of plant proteins and the like.

The membrane-forming material of the fluoro-carbon emulsion enhancement agent for HIFU treatment according to the present invention is preferably a biocompatible and degradable biomaterial, such as a lipid, such that the enhancement agent can be injected intravenously, transported through the blood circulation smoothly, and then phagocytosed quickly by the tissues of the human body, which are full of reticuloendothelial cells. Therefore, a mass of enhancement agent can be deposited in the tissues of the human body in a certain time, significantly changing the acoustic environment of the target tissue. Thus, the ultrasound absorption capacity of the tissue can be significantly enhanced, the acoustic energy deposition at the target tissue during HIFU treatment can be increased, and eventually the effectiveness with which clinical HIFU treatment can ablate tumor cells will be improved greatly.

In the above embodiment of the present invention, the liquid that undergoes a liquid-gas phase transition at 38-100°C includes C₅-C₆ alkanes, such as n-pentane, and i-pentane, and C₅-C₁₂ fluorohydrocarbons , and the like (see pages 65-70 of Chinese Patent No. ZL94191564 (Application No. CN1068230C)).

In the above embodiment of the present invention, the aqueous medium is distilled water, physiological saline or glucose solution. The concentration of the glucose solution can be up to 50% (w/v). But the glucose solution cannot be used as the aqueous medium for the fluoro-carbon emulsion enhancement agent for HIFU treatment in diabetic patients.

In a preferred embodiment, the enhancement agent may contain an emulsifier. The emulsifier is typically selected from a group consisting of ethylene glycol mono-C₁₆₋₁₈-fatty acid esters, diethylene glycol monO-C₁₆₋₁₈-fatty acid esters, diethylene glycol di-C₁₆₋₁₈-fatty acid esters, triethylene glycol mono-C₁₆₋₁₈-fatty acid esters, sorbitan fatty acid ester (Span type) emulsifiers, polysorbate (Tween type) emulsifiers, polyethylene glycol monolaurate-based emulsifiers, polyoxyethylene laurate-based emulsifiers, 3-sn-phosphatidylcholine (lecithin), cholic acid, and the like. The amount of the emulsifier in the enhancement agent is 5-150g/L. In addition, the enhancement agent may also contain a stabilizing agent, such as carboxymethylcellulose sodium (CMC-Na), carboxymethylcellulose potassium, carboxyethylcellulose sodium, carboxyethylcellulose potassium, carboxypropylcellulose sodium, carboxypropylcellulose potassium, glycerin, and the like. The amount of the CMC-Na contained in the enhancement agent is 0.01-10g/L, preferably 0.05-0.6g/L, and more preferably 0.1-0.3g/L. The amount of the glycerin contained in the enhancement agent is 5-100g/L.

In a more preferred embodiment, in order to increase the stability of the enhancement agent, the enhancement agent is adjusted to pH 7.0-9.0, preferably 7.5-8.5. Inorganic or organic acids or bases may be used to adjust the pH value of the enhancement agent.

Additionally, in order to make the fluoro-carbon emulsion enhancement agent for HIFU treatment according to the present invention target a specific tumor tissue or focus, substances having specific affinity to the tumor tissue or the focus, such as a tumor-specific antibody, may be added to the enhancement agent.

The fluoro-carbon emulsion ultrasound contrast agents that are widely used in ultrasound imaging may be used as the fluoro-carbon emulsion enhancement agent for HIFU treatment of the present invention. Thus, in another aspect, the present invention provides use of fluoro-carbon emulsion ultrasound contrast agent for preparing the enhancement agent for HIFU treatment.

In another embodiment, the present invention provides a method for preparing the fluoro-carbon emulsion enhancement agent for HIFU treatment. The method comprises:
(1) weighing and mixing a membrane-forming material and a core material to obtain 0.1-100g/L membrane-forming material and 5-200g/L core material, adding an aqueous medium to the mixture up to a predetermined volume, and stirring the mixture to form a coarse emulsion;
(2) emulsifying the coarse emulsion prepared in step (1) in a high pressure homogenizer (see Example 19 described in Chinese patent No. CN1068230C). Preferably, the coarse emulsion is emulsified twice.

In the method for preparing the fluoro-carbon emulsion enhancement agent for HIFU treatment of the present invention, the mixture is preferably stirred and dispersed in an ice bath in the above-mentioned step (1), and more preferably, an emulsifier and/or stabilizing agent may be added to the mixture when the membrane-forming material and core material are mixed.

The present invention is further directed to a method for increasing energy deposition at the target location during HIFU treatment, wherein, the method comprises administering an effective dosage of the fluoro-carbon emulsion enhancement agent of the present invention intravenously via continuous and rapid IV instillation or bolus injection to a patient at 0-30 minutes before applying HIFU treatment to a patient. The effective dosage mentioned above varies with the type of tumor, weight of patient, location of tumor, volume of tumor. However, a doctor or a pharmacist can easily determine the suitable dosage for different patients. For example, the dosage can be selected from the range of 0.005-0.1ml/kg, preferably 0.01-0.05ml/kg

### DETAILED DESCRIPTION OF THE INVENTION

### Example 1

The following materials were mixed to a final volume of 1000ml: 3% (w/v) emulsifier Pluronic F-68 (purchased from Sigma Company), 0.5% (w/v) yolk lecithin (purchased from Shanghai Chemical Reagent Company), 5% (v/v) perfluoropentane (purchased from Sigma Company), and distilled water. The mixture was incubated on ice and sheared and dispersed at 10000 rpm for 5 minutes to obtain a coarse emulsion. The coarse emulsion was emulsified in a high-pressure homogenizer at 4°C twice. The resulting emulsion with a particle size of less than 1µm was obtained by filtering through a 1µm membrane filter. The final emulsion was divided and put into 15ml vials, and then was radiated by Co₆₀ at 20KGY for 10 hours. The emulsion had a particle concentration of 10⁹/ml and was refrigerated for storage.

### Example 2

The following materials were mixed to a final volume of 1000ml: 6% (w/v) emulsifier Pluronic F-68 (purchased from Sigma Company), 1% (w/v) yolk lecithin (purchased from Shanghai Chemical Reagent Company), 10% (v/v) perfluoropentane (purchased from Sigma Company), and physiological saline solution. The mixture was incubated on ice and sheared and dispersed at 10000 rpm for 5 minutes to obtain a coarse emulsion. The coarse emulsion was emulsified in a high-pressure homogenizer at 4 °C twice. The resulting emulsion with a particle size of less than 1µm was obtained by filtering through a 1µm membrane filter. The final emulsion was divided and put into 15ml vials, and then was radiated by Co₆₀ at 20KGY for 10 hours. The emulsion had a particle concentration of of 10⁹/ml and was refrigerated for storage.

### Example 3-6

The fluoro-carbon emulsion enhancement agents for HIFU treatment of the present invention were prepared according to the same method and procedures described in Example 1 with the materials and the amounts thereof set forth in Table 1. The parameters of the products are shown in Table 1.

**Table 1**

| | Example 3 | Example 4 | Example 5 | Example 6 |
|---|---|---|---|---|
| Core material | 2% (v/v) Perfluoropentane | 5% (v/v) Perfluorohexane | 10% (v/v) Perfluorohexane | 10% (v/v) Dihydrodecafluoropentane |
| Lecithin | 1% (w/v) | 2% (w/v) | 2% (w/v) | 2% (w/v) |
| Glycerin | 1% (w/v) | 1% (w/v) | 1% (w/v) | 1% (w/v) |
| Pluronic F-68 | 5% (w/v) | 3% (w/v) | 5% (w/v) | 5% (w/v) |
| Final volume after distilled water added | 1000ml | 1000ml | 1000ml | 1000ml |
| PH (c.a.) | 6.98 | 7.01 | 6.99 | 7.00 |
| Particle size of the discontinuous phase | 0.5-2µm | 0.5-2µm | 0.1-2µm | 1-2µm |

The effects of the enhancement agent for HIFU treatment of the present invention are demonstrated below through the combined use of the fluoro-carbon emulsion enhancement agent for HIFU treatment as prepared in Example 1 and HIFU Tumor Therapeutic System Model-JC.

### Animal test 1 Study on lesions of New Zealand white rabbit liver

Twenty New Zealand white rabbits weighing 2.21±0.56kg, which were provided by the Laboratory Animals Center of Chongqing University of Medical Sciences, were used. These rabbits were shaved at the lower bosom and the midsection on the day prior to study. A High-intensity Focused Ultrasound Tumor Therapeutic System Model-JC manufactured by Chongqing Haifu (HIFU) Technology Co. Ltd. was used to radiate these rabbits. The High-intensity Focused Ultrasound Tumor Therapeutic System Model-JC was composed of an adjustable power generator, a B-mode ultrasound monitoring system, a therapeutic transducer, a mechanical motion control system, a treatment bed and an acoustic coupling device. The therapeutic transducer of the System, with working frequency of 1MHz, diameter of 150mm, and focal distance of 150mm, using standard circulating degassed water with gas content of less than or equal to 3ppm, can produce a focal region of 2.3x2.4x26mm and deliver an average acoustic intensity of 5500W/cm². The transducer used in this study was 150mm in diameter, and it had a focal distance of 135mm, a working frequency of 1.0MHz and an acoustic power of 200W. The exposure depth was 20mm, and the discontinuous single pulse exposure with exposure duration of 3s and interval of 5s was applied. The physiological saline solution (0.02ml/kg) was rapidly delivered via rabbit ear border vein to each rabbit, and the rabbit liver was exposed to HIFU using single pulse exposure 60 seconds later on the control side. The enhancement agent for HIFU treatment as prepared in Example 1 (0.02ml/kg) was rapidly delivered via rabbit ear border vein to each rabbit, and the other plane of the same rabbit liver of the control side were exposed with HIFU at 60 seconds later for the experimental side. The ultrasound exposures finished when gray-scale changes occurred at the target location. If there is no gray-scale change to be seen, the total exposure duration should be no more than 20s. Three days after ultrasound exposure, the rabbits were sacrificed by breaking the necks and were dissected. The volume (V) of coagulative necrosis of rabbit liver was measured. The EEF was calculated according to the expression *EEF* = ^{η*Pt*}/*_{V}*, wherein, t refers to the exposure time and η=0.7. The median of the EEFs was 6.0160 on the control side and 1.2505 on the experimental side. Wilcoxon signed rank-sum test showed Z=-2.485, and P=0.013. The results of this study show that the fluoro-carbon emulsion increases the effectiveness of HIFU to cause lesions of the rabbit livers. In fact, the mean of the EEF in the control side is 4.81 times as much as the mean of the EEF in the experimental side.

### Animal test 2 Study on lesions of goat liver

Twenty Nanjiang yellow goats weighing 22.25±4.51kg were used. The goats were shaved at the right bosom and the right abdomen on the day of the study. A High-intensity Focused Ultrasound Tumor Therapeutic System Model-JC manufactured by Chongqing Haifu (HIFU) Technology Co. Ltd. was used to radiate these yellow goats. The transducer used in this study was 150mm in diameter, and it had a focal distance of 135mm, a working frequency of 0.8MHz and an acoustic power of 220W. The exposure depth was 30mm, and a discontinuous single pulse exposure with exposure duration of 3s and interval of 5s was applied. Ribs of all the goats were not removed. A pre-scanning was carried out before HIFU exposure and the areas for exposure including 4 planes were selected. One exposure spot was introduced on each plane, and two-dimensional ultrasound was used to monitor the rib clearance. The physiological saline solution (0.02ml/kg) was rapidly delivered intravenously via ear border to each goat, and the goat liver was exposed to HIFU 60 seconds later and two exposure spots were introduced on each goat on the control side. The enhancement agent for HIFU treatment as prepared in Example 1 (0.02ml/kg) was rapidly delivered intravenously via ear border to each goat, and the goat liver was exposed to HIFU 60 seconds later and two exposure spots were introduced on each goat on the experimental side. When gray-scale changes occurred at the target location, the exposures were repeated another 4 or 5 times. If there is no gray-scale change to be seen, the total exposure duration should be no more than 200s. Three days after ultrasound exposure, the goats were sacrificed and dissected. The volume (V) of coagulative necrosis of goat liver was measured. The EEF was calculated according to the expression *EEF* = ^{η*Pt*}/*_{V}*, wherein, T refers to the exposure time and η=0.7. The median of EEFs was infinite for the control side and 5.1904 for the experimental side using the combination of HIFU and fluoro-carbon emulsion. Wilcoxon signed rank-sum test showed P=0.004. This study shows that the fluoro-carbon emulsion significantly increased the ability of HIFU to create lesions in goat livers without removal of the ribs of the goats.

### Animal test 3 Study on lesions of goat kidney

Twenty Nanjiang yellow goats weighing 22.25±4.51kg were used. These goats were shaved at the right bosom and the right abdomen on the day of the study. A High-intensity Focused Ultrasound Tumor Therapeutic System Model-JC manufactured by Chongqing Haifu (HIFU) Technology Co. Ltd. was used to radiate these yellow goats. The transducer used in this study was 150mm in diameter, and it had a focal distance of 135mm, a working frequency of 0.8MHz and an acoustic power of 220W. The exposure depth was 20mm, and a discontinuous single pulse exposure with exposure duration of 3s and interval of 5s was applied. Ribs of all the goats were not removed. A pre-scan was carried out before HIFU exposure and the areas for exposure including 1 plane on the upper pole of kidney and 1 plane on the lower pole of kidney respectively were selected. One exposure spot was introduced on each plane, and two-dimensional ultrasound was used for observation. The right ribs were avoided if they become obstacles. The physiological saline solution (0.02ml/kg) was rapidly delivered intravenously via ear border to each goat, and the goat kidney was exposed to HIFU under single pulse exposure 30 seconds later on the control side. The enhancement agent for HIFU treatment as prepared in Example 1 (0.02ml/kg) was delivered rapidly intravenously via ear border to each goat, and the goat kidney was exposed to HIFU 60 seconds later for the experimental side. When gray-scale changes occurred at the target location, the exposures were repeated another 3 or 4 times. If there is no gray-scale change to be seen, the total exposure duration should be no more than 150s. Three days after ultrasound exposure, the goats were sacrificed and dissected. The volume (V) of coagulative necrosis of goat kidney was measured. The EEF was calculated according to the expression *EEF* = ^{η*Pt*}/*_{V}*, wherein, T refers to the exposure time and η=0.7. The EEFs were 10.58±3.95 for the experimental side and 486.37±215.41 for the control side. Wilcoxon signed rank-sum test showed P=0.008. The results of this study indicate that the fluoro-carbon emulsion greatly increased the ability of HIFU to cause lesions in normal goat kidneys. In fact, the mean of the EEF in the control side is more than 40 times the mean of the EEF in the experimental side.

### INDUSTRIAL APPLICABILITY

The fluoro-carbon emulsion enhancement agent for HIFU treatment of the present invention can change the acoustic environment of the target location greatly and can reduce the acoustic energy needed to cause lesions of a target tissue (tumor and non-tumor tissue) per unit volume of the tissue during HIFU treatment. Accordingly, deep-seated and large-sized tumors can be treated with HIFU treatment more effectively under a certain acoustic power without damaging the normal tissue along the acoustic pathway. It becomes possible to use the enhancement agent for HIFU treatment of the present invention to effectively treat patients with hepatic tumors that are blocked by the ribs without removal of the ribs.

## Claims

1. An enhancement agent for use in high intensity focused ultrasound (HIFU) treatment, wherein, the enhancement agent comprises a discontinuous phase comprised of a core material encapsulated by a membrane-forming material, and a continuous phase comprised of aqueous medium, the discontinuous phase is uniformly dispersed in the continuous phase and the particle size of the discontinuous phase ranges from 0.1-2µm; the amount of the membrane-forming material in the enhancement agent is 0.1-100g/L; the core material is comprised of a liquid that undergoes a liquid-gas phase transition at 38-100°C, and the amount of the core material in the enhancement agent is 5-200ml/L

2. The enhancement agent according to claim 1, wherein the discontinuous phase has a particle size ranging from 0.1-1µm.

3. The enhancement agent according to claim 1, wherein the membrane-forming material is one or more substances selected from a group consisting of phospholipin, cholesterol and glycolipide.

4. The enhancement agent according to claim 3, wherein the membrane-forming material comprises phospholipid selected from a group consisting of 3-sn-phosphatidylcholine, 1,2-dipalmitoyl-sn-glycero-3-phosphatidylglycerol sodium salt, 1,2-distearoyl-sn-glycero-3-phosphatidylcholine, sodium 1,2-dipalmitoyl-sn-glycero-3-phosphatidate, 1,2-dipalmitoyl-sn-glycero-3-phosphatidylcholine, phosphatidylserine and hydrogenated phosphatidylserine.

5. The enhancement agent according to claim 1, wherein the amount of the membrane-forming material in the enhancement agent is 1-50g/L.

6. The enhancement agent according to claim 5, wherein the amount of the membrane-forming material in the enhancement agent is 5-20g/L.

7. The enhancement agent according to claim 1, wherein the core material is one or more substances selected from the group consisting of C₅-C₆ alkanes and Cₛ-C j2 fluorohydrocarbons.

8. The enhancement agent according to claim 7, wherein the core material is dihydrodecafluoropentane.

9. The enhancement agent according to claim 1, wherein the amount of the core material in the enhancement agent is 10-100ml/L.

10. The enhancement agent according to claim 9, wherein the amount of the core material in the enhancement agent is 20-80ml/L.

11. The enhancement agent according to claim 1, wherein the aqueous medium is comprised of distilled water, physiological saline solution or glucose solution.

12. The enhancement agent according to any one of claims 1-11, wherein the enhancement agent contains an emulsifier in an amount of 5-150g/L, the emulsifier is selected from a group consisting of ethylene glycol mono-C₁₆₋₁₈-fatty acid esters, diethylene glycol mono-C₁₆₋₁₈-fatty acid esters, diethylene glycol di-C₁₆₋₁₈-fatty acid esters, triethylene glycol mono-C₁₆₋₁₈-fatty acid esters, sorbitan fatty acid esters, polysorbate, polyethylene glycol monolaurate, polyoxyethylene laurate, 3-sn-phosphatidylcholine, polyethylene oxide/polypropylene oxide/ethylene glycol non-ionic block copolymer, fluorosurfactant, and cholic acid.

13. The enhancement agent according to any one of claims 1-11, wherein the enhancement agent contains a stabilizing agent comprising carboxymethylcellulose sodium; the amount of the carboxymethylcellulose sodium in the enhancement agent is 0.01-10g/L

14. The enhancement agent according to any one of claims 1-11, wherein the enhancement agent contains a stabilizing agent comprising glycerin; the amount of the glycerin in the enhancement agent is 5-100g/L.

15. Use of a fluoro-carbon emulsion ultrasound contrast agent according to any one of claims 1-14 for the preparation of a medicament for HIFV treatment.

## Patentansprüche

1. Verstärkungsmittel zur Verwendung bei fokussierter Hochintensitäts-Ultraschallbehandlung (high intensity focused ultrasound = HIFU) wobei das Verstärkungsmittel eine diskontinuierliche Phase, die sich aus einem von einem membranbildenden Material eingekapselten Kernmaterial zusammensetzt, und eine kontinuierliche Phase umfasst, die sich aus einem wässrigen Medium zusammensetzt; die diskontinuierliche Phase ist in der kontinuierlichen Phase gleichmäßig dispergiert und die Teilchengröße der diskontinuierlichen Phase reicht von 0,1 bis 1µm; die Menge des membranbildenden Materials in dem Verstärkungsmittel beträgt 0,1 bis 100 g/L; das Kernmaterial ist zusammengesetzt aus einer Flüssigkeit, die bei 38 - 100°C einen Flüssigkeits-Gas-Phasenübergang durchmacht und die Menge des Kernmaterials im Verstärkungsmittel beträgt 5 - 200 ml/L.

2. Verstärkungsmittel nach Anspruch 1, wobei die diskontinuierliche Phase eine Teilchengröße hat, die von 0,1 bis 0,5µm reicht.

3. Verstärkungsmittel nach Anspruch 1, wobei das membranbildende Material aus einer oder mehreren Substanzen besteht, die ausgewählt werden aus einer Gruppe, die aus Phospholipin, Cholesterol und Glycolipid besteht.

4. Verstärkungsmittel nach Anspruch 3, wobei das membranbildende Material Phospholipid umfasst, das aus einer Gruppe ausgewählt wird, die aus 3-sn-Phosphatidylcholin, 1,2-Dipalmitoyl-sn-Glycero-3-Phosphatidylglycerol Natriumsalz, 1,2-Distearoyl-sn-Glycero-3-Phosphatidylcholin, Natrium 1,2-Dipalmitoyl-sn-Glycero-3-Phosphatidase, 1,2-Dipalmitoyl-sn-Glycero-3-Phosphatidylcholin, Phosphatidylserin und hydriertem Phosphatidylserin besteht.

5. Verstärkungsmittel nach Anspruch 1, wobei die Menge des membranbildenden Materials in dem Verstärkungsmittel 1 - 50 g/L beträgt.

6. Verstärkungsmittel nach Anspruch 5, wobei die Menge des membranbildenden Materials in dem Verstärkungsmittel 5 - 20 g/L beträgt.

7. Verstärkungsmittel nach Anspruch 1, wobei das Kernmaterial aus einer oder mehreren Substanzen besteht, die ausgewählt werden aus einer Gruppe, die aus C₅-C₆ Alkanen und C₅-C₁₂ Fluorkohlenwasserstoffen besteht.

8. Verstärkungsmittel nach Anspruch 7, wobei das Kernmaterial Dihydrodekafluorpentan ist.

9. Verstärkungsmittel nach Anspruch 1, wobei die Menge des Kernmaterials in dem Verstärkungsmittel 10 - 100 ml/L beträgt.

10. Verstärkungsmittel nach Anspruch 9, wobei die Menge des Kernmaterials in dem Verstärkungsmittel 20 - 80 ml/L beträgt.

11. Verstärkungsmittel nach Anspruch 1, wobei das wässrige Medium zusammengesetzt ist aus destilliertem Wasser, physiologischer Salzlösung oder Glukoselösung.

12. Verstärkungsmittel nach einem der Ansprüche 1-11,wobei das Verstärkungsmittel einen Emulgator in einer Menge von 5 - 150 g/L enthält, der Emulgator ausgewählt wird aus einer Gruppe, die aus Ethylenglykolmono-C₁₆₋₁₈ fettigen Säureestern, Diethylenglykolmono-C₁₆₋₁₈ fettigen Säureestern, Diethylenglykoldi-C₁₆₋₁₈ fettigen Säureestern, Triethylenglykolmono-C₁₆₋₁₈ fettigen Säureestern, Sorbitan fettigen Säureestern, Polysorbat, Polyethylenglykolmonolaurat, Polyoxyethylenlaurat, 3-sn-Phosphatidylcholin, Polyethylenoxid/polypropylenoxid /Ethylenglykol nichtionisches Blockcopolymer Fluortensid und Cholsäure besteht.

13. Verstärkungsmittel nach einem der Ansprüche 1-11, wobei das Verstärkungsmittel ein Stabilisierungsmittel enthält, das Carboxymethylcellulosenatrium umfasst; die Menge des Carboxymethylcellulosenatrium in dem Verstärkungsmittel beträgt 0,01 - 10 g/L.

14. Verstärkungsmittel nach einem der Ansprüche 1-11, wobei das Verstärkungsmittel ein Stabilisierungsmittel enthält, das Glycerin umfasst; die Menge des Glycerins in dem Verstärkungsmittel beträgt 5 - 100 g/L.

15. Verwendung eines Flourkohlenstoff-Emulsions-Ultraschallkontrastmittels nach einem der Ansprüche 1-14 für die Herstellung eines Medikaments zur HIFU Behandlung.

## Revendications

1. Agent d'amplification destiné à être utilisé dans un traitement ultrasonique focalisé à haute intensité (HIFU), l'agent d'amplification comprenant une phase discontinue constituée d'une matière centrale encapsulée par une matière de formation de membrane, et une phase continue constituée d'un milieu aqueux, la phase discontinue étant dispersée uniformément dans la phase continue et le diamètre de particule de la phase discontinue allant de 0,1 à 1 µm ; la quantité de la matière de formation de membrane dans l'agent d'amplification étant de 0,1 à 100 g/l ; la matière centrale étant constituée d'un liquide qui subit une transition de phase liquide-gaz à 38-100°C, et la quantité de la matière centrale dans l'agent d'amplification étant de 5 à 200 ml/l.

2. Agent d'amplification suivant la revendication 1, dans lequel la phase discontinue a un diamètre de particule compris dans l'intervalle de 0,1 à 0,5 µm.

3. Agent d'amplification suivant la revendication 1, dans lequel la matière de formation de membrane consiste en une ou plusieurs substances choisies dans un groupe consistant en un phospholipide, le cholestérol et un glycolipide.

4. Agent d'amplification suivant la revendication 3, dans lequel la matière de formation de membrane comprend un phospholipide choisi dans un groupe consistant en la 3-sn-phosphatidylcholine, le sel de sodium de 1,2-dipalmitoyl-sn-glycéro-3-phosphatidylglycérol, la 1,2-distéaroyl-sn-glycéro-3-phosphatidylcholine, le 1,2-dipalmitoyl-sn-glycéro-3-phosphatidate de sodium, la 1,2-dipalmitoyl-sn-glycéro-3-phosphatidylcholine, la phosphatidylsérine et la phosphatidylsérine hydrogénée.

5. Agent d'amplification suivant la revendication 1, dans lequel la quantité de la matière de formation de membrane dans l'agent d'amplification est de 1 à 50 g/l.

6. Agent d'amplification suivant la revendication 5, dans lequel la quantité de la matière de formation de membrane dans l'agent d'amplification est de 5 à 20 g/l.

7. Agent d'amplification suivant la revendication 1, dans lequel la matière centrale consiste en une ou plusieurs substances choisies dans le groupe consistant en des alcanes en C₅ et C₆ et des fluorohydrocarbures en C₅ à C₁₂.

8. Agent d'amplification suivant la revendication 7, dans lequel la matière centrale est le dihydrodécafluoro-pentane.

9. Agent d'amplification suivant la revendication 1, dans lequel la quantité de la matière centrale dans l'agent d'amplification est de 10 à 100 ml/l.

10. Agent d'amplification suivant la revendication 9, dans lequel la quantité de la matière centrale dans l'agent d'amplification est de 20 à 80 ml/l.

11. Agent d'amplification suivant la revendication 1, dans lequel le milieu aqueux est constitué d'eau distillée, d'une solution saline physiologique ou d'une solution de glucose.

12. Agent d'amplification suivant l'une quelconque des revendications 1 à 11, ledit agent d'amplification contenant un émulsionnant en une quantité de 5 à 150 g/l, l'émulsionnant étant choisi dans un groupe consistant en des mono-esters d'acides gras en C₁₆ à C₁₈ d'éthylèneglycol, des mono-esters d'acides gras en C₁₆ à C₁₈ de diéthylèneglycol, des di-esters d'acides gras en C₁₆ à C₁₈ de diéthylèneglycol, des mono-esters d'acides gras en C₁₆ à C₁₈ de triéthylèneglycol, des esters d'acides gras de sorbitane, un polysorbate, le monolaurate de polyéthylèneglycol, le laurate de polyoxyéthylène, la 3-sn-phosphatidylcholine, un copolymère séquencé non ionique poly(oxyde d'éthylène)/poly-(oxyde de propylène)/éthylèneglycol, un agent tensioactif fluoré et l'acide cholique.

13. Agent d'amplification suivant l'une quelconque des revendications 1 à 11, ledit agent d'amplification contenant un stabilisant comprenant de la carboxyméthylcellulose sodique ; la quantité de carboxyméthylcellulose sodique dans l'agent d'amplification étant de 0,01 à 10 g/l.

14. Agent d'amplification suivant l'une quelconque des revendications 1 à 11, ledit agent d'amplification contenant un stabilisant comprenant du glycérol, la quantité de glycérol dans l'agent d'amplification étant de 5 à 100 g/l.

15. Utilisation d'un agent de contraste ultrasonique en émulsion à base fluorocarbonée suivant l'une quelconque des revendications 1 à 14, pour la préparation d'un médicament destiné à un traitement HIFU.
